# EUROPEAN PATENT APPLICATION

(11) **EP 2 399 552 A1**
(43) Date of publication of application: **28.12.2011**
(21) Application number: 10167082.6
(22) Date of filing: 23.06.2010
(51) Int. Cl.: A61F 2/90

(54) **Stent and method for implanting said stent**

(71) Applicant: Lanzer, Peter, 06869 Coswig/Anhalt (DE)
(72) Inventor: Lanzer, Peter, 06869 Coswig/Anhalt (DE)
(74) Representative: Peckmann, Ralf

(57) **Abstract**

The present invention relates to a stent (8), in particular for the treatment of cardiovascular diseases, wherein an end (12) of said stent (8) comprises a slant (17). The present invention further relates to a method for implanting such a stent (8), comprising the following method steps: introducing said stent (8) into a side branch vessel (3) which branches from a parent vessel (1); and arranging said slant (17) of said stent (8) in such a way that said slant (17) is oriented towards a branching point (5) of said vessels (1, 3) and said slant (17) is flush with an inner surface (23) of said parent vessel (1).

## Description

The present invention relates to a stent and to a method for implanting such a stent.

The number of coronary heart diseases consistently rises in industrialized countries. One widespread method of treating such coronary heart diseases are so-called percutaneous coronary intervention (PCI) methods. Thereby, a disease-induced flow restriction in a vessel is removed by dilating the blocked vessel by means of an inflatable balloon, for example. PCI normally induces injuries in the walls of the treated vessels. These injuries could lead to a recoil, collapse or restenosis of the vessel in the treated area. Therefore, after or during dilating the vessel which could be an artery or a vein, often a so-called stent is implanted into the treated vessel to keep it open and/or to reinforce it.

Typically, a stent is a metallic mesh in the form of a hollow cylinder. The stent is introduced into the body in an unexpanded state and is expanded at the site where the stent should be placed to its final diameter. The stent thus acts as a scaffold that reinforces the wall of the vessel. Vessels in their course branch or bifurcate like arteries or flow together like veins. Typically, the angulations between a parent vessel and a side branch that leads into the parent vessel range widely, e.g. in human coronary arteries between 32° and 124°. Vascular lesions are frequently located at this branching or bifurcation points, e.g. up to 20% of all coronary artery lesions treated by means of PCI.

Due to the angulations it is not possible to place a stent in the area of a bifurcation point into the branch vessel without at least partly extending into the lumen of the parent vessel. This is very disadvantageous concerning an undisturbed blood flow and increases the risk of thrombosis or restenosis. This problem is even intensified when it is necessary to implant a further stent into the parent vessel in the area of the branching point of the vessels. Therefore, up to now it is unavoidable to modify and to deform the hollow cylinder shaped stents after implantation to adapt to the conditions at the branching point. This modification is for example done by the multiple use of balloon catheters.

Due to the shortcomings of the currently available revascularization techniques the clinical outcome of endovascular repair of branching and bifurcation lesions is associated with a significantly higher incidence of complications such as technical failure, stent thrombosis and restenosis.

It is therefore an object of the present invention to provide an improved stent for the treatment of cardiovascular diseases.

This object is achieved by a stent according to claim 1 and/or a by method according to claim 11 of the present invention.

Accordingly, a stent, in particular for the treatment of cardiovascular diseases is provided, wherein an end of said stent comprises a slant.

Further, a method for implanting such a stent is provided, comprising the following method steps: introducing said stent into a side branch vessel which branches from a parent vessel; and arranging said slant of said stent in such a way that said slant is oriented towards a branching point of said vessels and said slant is flush with an inner surface of said parent vessel.

The basic idea of the present invention is to provide a slant at one end of the stent.

Consequently, it is possible to arrange the slant in such a way that the stent does not protrude into the parent vessel and is flush with the parent vessels inner surface. This reduces the danger of thrombosis and restenosis and enables an undisturbed flow of blood through the treated area.

Advantageous embodiments and configurations of the invention result from the dependent claims and from the description in synopsis with the figures of the drawing.

In an embodiment of the present invention said slant truncates a cylindrical stent shape which is generated by a lateral surface of said stent at least sectionally. This securely leads to a slant geometry that does not protrude into the parent vessel when the stent is implanted.

In an advantageous embodiment of the present invention said slant comprises a slant angle disposed between a face side and a slant plane of said stent and said slant comprises a slant length disposed along a longitudinal axis of said stent defining a distance between said face side and an intersection point between said slant plane and said lateral surface of said stent. This enables an accurate determination of the slant geometry which simplifies the effort to produce said slant.

In a typical embodiment of the present invention said slant angle is between 10° and 60°. With this angle range typical physiological side branch angles are comfortably covered.

In another advantageous embodiment of the present invention said slant is designed in such a way that when implanting said stent into a side branch vessel which branches from a parent vessel under a branching angle, said slant is oriented towards a branching point of said vessels and said end of said stent which comprises said slant is flush with an inner surface of said parent vessel and said slant scaffolds an ostial circumference and a lumen of said branch vessel. This prevents the slanted end of the stent dependably from expanding into the lumen of the parent vessel.

In another advantageous embodiment of the present invention said stent is a balloon-expandable or a self-expanding stent. This enables an easy and comfortable application of the stent.

In a further advantageous embodiment of the present invention said stent comprises a mesh-like structure comprising a first radiopaque marker being disposed at a most proximal mesh cell of said stent and/or a second radiopaque marker being disposed at a most distal mesh cell of said stent. By means of the preferably two radiopaque markers the slanted end of the stent is easily visible under X-rays, for example, which simplifies the application of the stent.

In a typical embodiment of the present invention, when implanting said stent into a side branch vessel said first radiopaque marker can be placed to match a most proximal point and/or said second radiopaque marker can be placed to match a most distal point of an ostial circumference of said branching vessels. This enables a very exact positioning of the stent inside the side branch vessel that has to be stented.

In another advantageous embodiment of the present invention said stent comprises a metal and/or a polymer material, said stent is of a bioabsorbable or a permanent type, and/or said stent comprises an open or covered stent design. Hereby, the properties of the stent are adjustable to the demands.

In a further embodiment of the present invention said stent is a drug-eluting stent. This helps to prevent restenosis, whereby the application area of the stent is comfortably widened.

In one embodiment of the present invention said stent is a balloon-expandable or a self-expanding stent and after arranging said slant said stent is expanded. This enables an easy and comfortable application of the stent.

In a preferred embodiment of the present invention said stent comprises a first radiopaque marker being disposed at a most proximal mesh cell of said stent and/or a second radiopaque marker being disposed at a most distal mesh cell of said stent, wherein during dilating and implanting said stent said first radiopaque marker is placed to match a most proximal point and/or said second radiopaque marker is placed to match a most distal point of an ostial circumference of said branching vessels. This enables an easy, comfortable and exact positioning of the slanted end of the stent at the branching point of the vessels.

In a further embodiment of the present invention a further stent is implanted into said parent vessel, in particular in the area of said branching point of said vessels. This enables the treatment of vascular lesions in the area of a branching point in both the branch and the parent vessel and thus widens the application possibilities of the method.

In another embodiment of the invention the longitudinal axes of said stents intersect each other. Due to the slanted end of the stent this is possible without modifying or damaging the stents at their contact area and thus simplifies their application.

The configurations and embodiments of the invention described above might be combined - if nothing else is explicated - complimentarily with each other.

In the following, exemplary embodiments of the invention will be explained in more detail with reference to the figures of the drawing, wherein:
- Fig. 1: shows a sectional view through a parent vessel and two branch vessels;
- Fig. 2: shows a three-dimensional partial view of a preferred embodiment of a stent with a large angulation angle;
- Fig. 3: shows a three-dimensional partial view of a further preferred embodiment of a stent with a small angulation angle;
- Fig. 4: shows a plane partial view of the stent according to Fig. 2;
- Fig. 5: shows a plane partial view of the stent according to Fig. 3; and
- Fig. 6: shows a sectional view through a vessel with implanted stents in the parent and branch vessels.

In the figures of the drawing, same structural elements have the same reference numerals if nothing else is explicated.

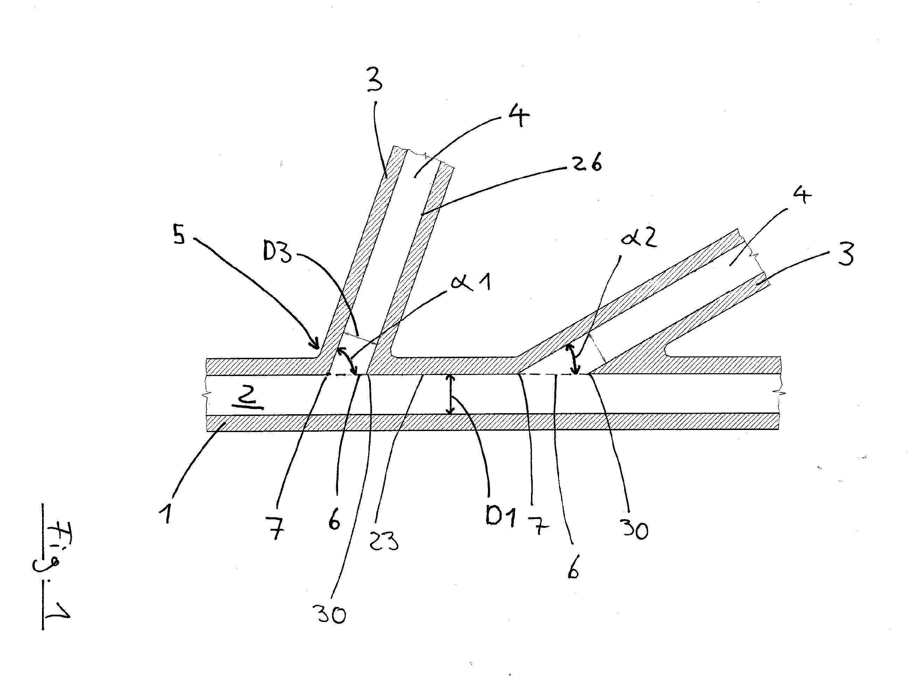

Figure 1 shows a sectional view through a middle axis of a parent vessel 1, for example a coronary or peripheral artery or vein. The parent vessel 1 has a lumen 2 in which a body fluid, in particular blood is transported. The lumen 2 has a diameter D1. The lumen 2 is defined by an inner surface 23 of the parent vessel 1. Branch vessels 3 branch or bifurcate from the parent vessel 1 under a branching or bifurcation angle a. The angle α is measured between an inner surface 26 of the branch vessel 3 and the inner surface 23 of the parent vessel 1. The inner surface 26 of the branch vessel 3 delimits a lumen 4 of the branch vessel 3. Depending on the parent vessel 1, the branch vessels 3 are arteries and veins, respectively. The branching or bifurcation angle α is known to be in a range of 32° to 124° in human coronary arteries, for example. Figure 1 shows the branch vessels 3 with two different branching angles α1, α2. The branch vessel 3 shown on the left hand side has an angle α1 of about 70° whereas the branch vessel 3 on the right hand side has a branching angle α2 of about 30°. The number of branch vessels 3 that bifurcate or branch from the parent vessel 1 is arbitrary. Also is the spatial orientation of the branch vessels 3 relatively to a longitudinal direction of the parent vessel 1. For simplification reasons, in the following reference is only made to one branch vessel 3. The lumen 4 of the branch vessel 3 has an inner diameter D3. The Branch vessel 3 and the parent vessel 1 intersect each other in a bifurcation or branching point 5, wherein an intersection curve of the inner surface 26 of the branch vessel 3 and the inner surface 23 of the parent vessel 1 is indicated as ostial circumference 6. On the ostial circumference 6 lies a most proximal point 7 and a most distal point 30 thereof. In the sectional view of the vessels 1, 3 the lumen 4 of the branch vessel 3 has the shape of a truncated cylinder or a truncated wedge, wherein the most proximal point 7 is disposed at the tip of this truncation and the most distal point 30 is disposed at the base of this truncation on the ostial circumference 6. After accomplishing PCI, at the branching point 5 often vascular injuries occur that have to be further treated.

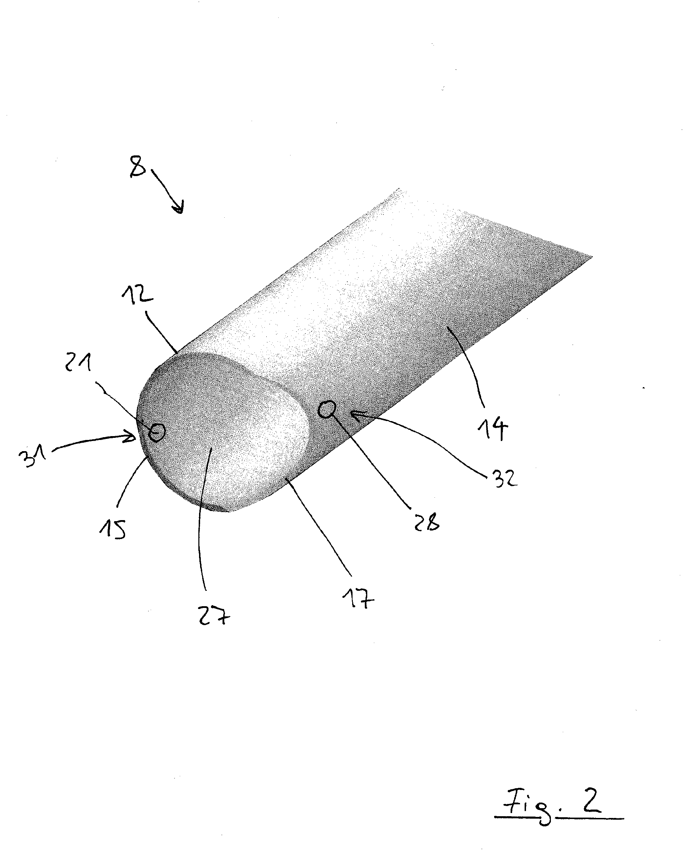

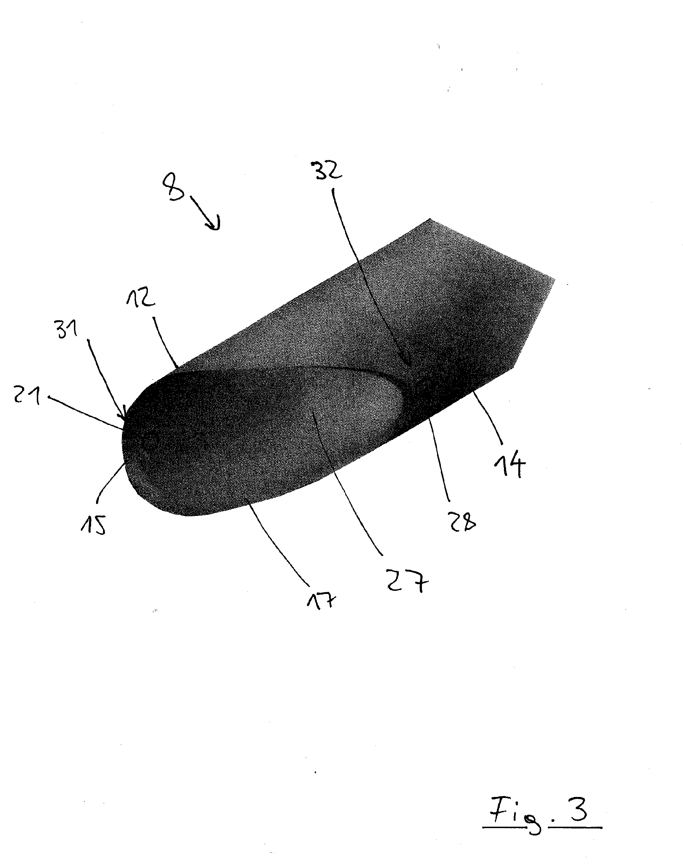

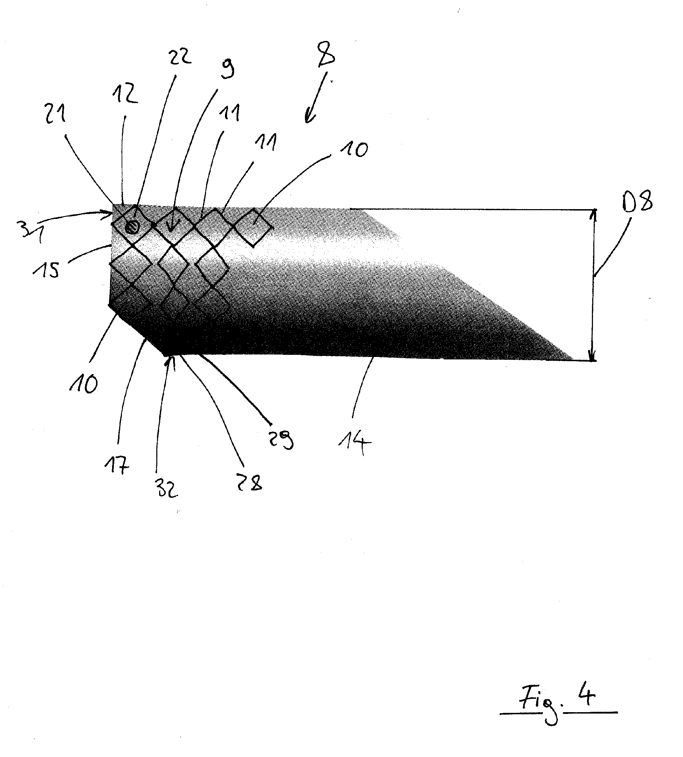

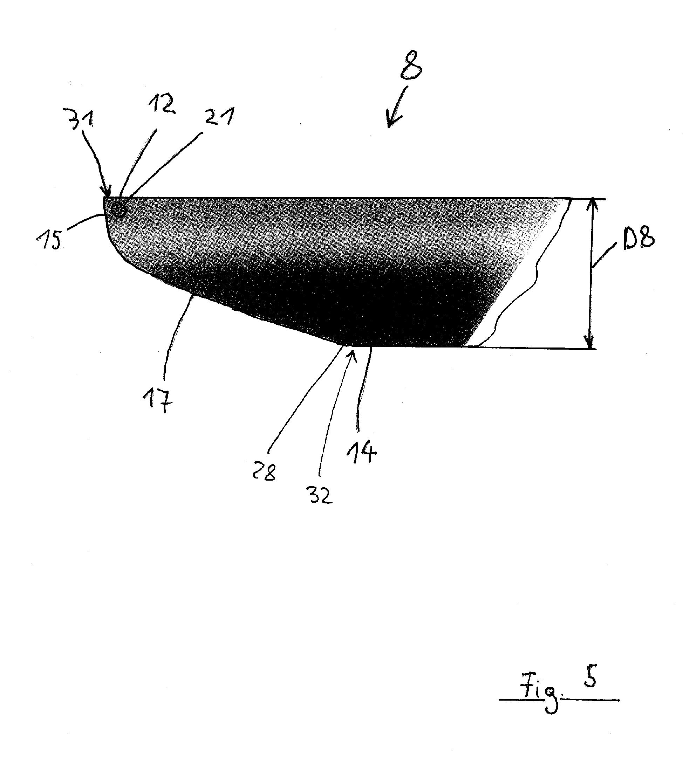

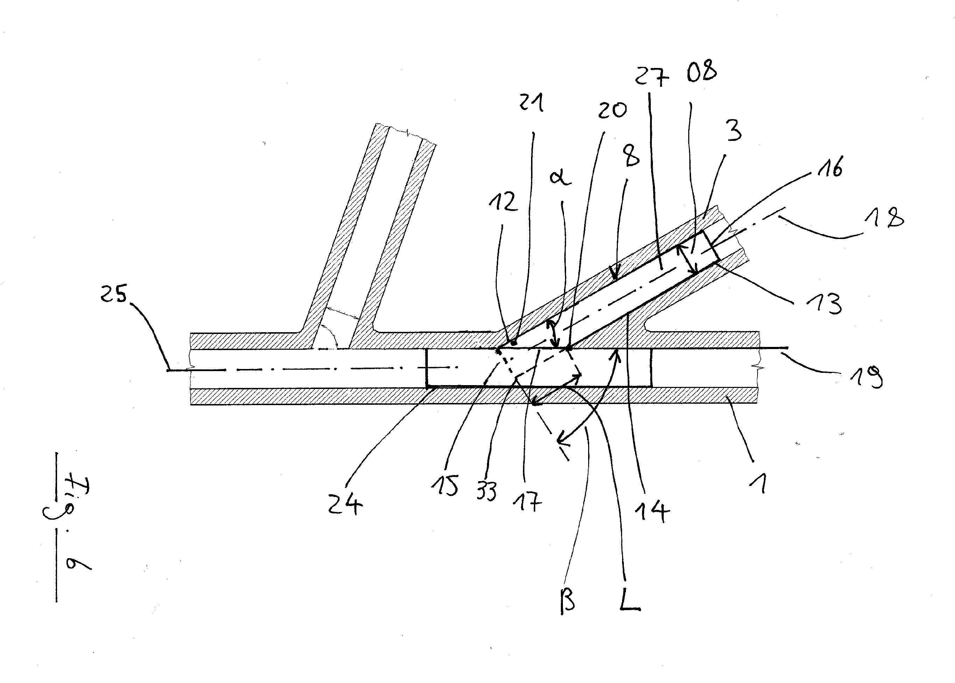

Figures 2 and 3 display three-dimensional views of preferred embodiments of a stent 8 and Figures 4 and 5 show plane views on these preferred embodiments of the stent 8. Figure 6 displays a preferred embodiment of the stent 8 implanted into a branch vessel 3. In the following, reference is made to Figures 2 to 6 at the same time.

The stent 8 has preferably the shape of a hollow cylinder with an inner cylindrical stent space 27 which is generated by a lateral surface 14 of said stent 8, wherein the cylinder wall of the stent 8 preferably consists of a mesh structure 9. Preferably, the inner cylindrical stent space 27 is defined by the lateral surface 14 of the stent and has the shape of a cylinder which is bordered by the lateral surface 14. For simplification, the mesh structure 9 is only shown in Fig. 4 and only throughout a part of the stent 8. The pattern of the mesh structure 9 is arbitrary and only shown for clarification reasons. The mesh structure 9 comprises so-called cells 10 which are separated from each other by means of so-called struts 11. The mesh structure 9 of the stent 8 is preferably designed such that the stent 8 is expandable from an initial state with an initial diameter to an expanded state with an expanded diameter D8. The diameter D8 preferably corresponds to the diameter D3 of the branch vessel 3. Figures 2 to 6 display the stent 8 in its expanded state. The mesh structure 9 is made by means of laser milling, for example. The stent 8 is preferably made of a metal alloy, more preferably of a steel alloy or a shape memory alloy like Nitinol. Alternatively, the stent 8 is made of a bioabsorbable material, in particular of a bioresorbable metal alloy or a bioresorbable polymer material. The stent 8 is for example provided with a polymer or ceramic coating. The stent 8 could be provided with drugs that elute from the stent 8 over a predetermined time to prevent restenosis. Further, the material of the stent 8 could be radioactively activated. In a preferred embodiment of the stent 8, the stent 8 has an open or a covered stent design. A longitudinal length of the stent 8 is almost arbitrary.

The stent 8 further comprises two end sections 12, 13 of the lateral surface 14. Each of the end sections 12, 13 has a face side 15, 16. Preferably one of the end sections 12, 13, named proximal end, is provided with a slant 17. The slant 17 preferably truncates the inner cylindrical stent space 27 which is generated by the lateral surface 14 and the lateral surface 14 itself at least sectionally. Thus, the stent 8 generally has the shape of a truncated hollow cylinder, wherein the truncation is provided at the slanted end section 12. Alternatively both end sections 12, 13 could comprise a slant 17, respectively, to fit a more complex branching pattern. The end section 12 with the slant 17 is preferably provided with a modified mesh structure 9 to allow a uniform expansion of the slanted end 12 when the stent 8 is expanded. For example when the stent 8 is expanded by means of a balloon. The modification of the mesh structure 9 may comprise cells 10 with a shape and/or size that differs from the shape and/or size of the cells 10 of the mesh structure 9 which is provided in the areas of the stent 8 without the slant 17. Figures 2 and 4 display an exemplary embodiment of the stent 8, wherein the slant 17 truncates just a part of the lateral surface 14 and the inner cylindrical stent space 27 which is generated by the lateral surface 14. This preferred embodiment of the stent 8 is preferably used with a branch vessel 3 with an bifurcation angle α of 70°. The slant 17 is preferably designed such that it is capable to scaffold both the ostial circumference 6 and the inner surface 26 of the branch vessel 3.

Figures 3 and 5 show an alternative exemplary embodiment of the stent 8, wherein the slant 17 truncates both the complete lateral surface 14 and the inner cylindrical stent space 27. This embodiment of the stent 8 according to Figures 3 and 5 is for example used with an bifurcation or branching angle α of 30°.

As displayed in Figure 6, the stent 8 has a longitudinal axis 18. The slant 17 forms a slant plane 19. Between this slant plane 19 and the face side 15 is measured a slant angle β. The slant plane 19 is also preferably defined as an intersection of two cylinders with different diameters representing the parent and branching vessels 1, 3. The slant angle β is preferably measured between the plane 19 which corresponds to a plane 19 of an ostium of the branching vessels 1, 3 and the face side 15. In the sectional view of Fig. 6 the face side 15 - illustrated by a dashed line - penetrates into the inner surface 23 of the parent vessel 1. The value of the slant angle β is preferably chosen in a range to accommodate the individual branching angle α of the vessels 1, 3. The slant angle β is also called stent-to-vessel angle because it describes an angle between the face side 15 and the plane 19 of the ostium of the branching vessels 1, 3. The slant angle β can be described as 90°-α. The slant 17 further comprises a slant length L. The slant length L is measured parallel to the longitudinal axis 18 between the face side 15, in particular between an distal edge 33 of the face side 15, and an intersection point 20 between the slant plane 19 and the lateral surface 14 of the stent 8. In other words, the slant length L is measured parallel to the longitudinal axis 18 between the distal edge 33 of the face side 15 and the intersection point 20 which is also an intersection point between the plane 19 of the ostial circumference 6 and a line drawn through the distal edge 33 of the face side 15 and the most distal point 30 of the ostial circumference 6. The slant length L is dependant on the slant angle β and can be calculated as L=tanβ*D8 with D8 being the diameter of the stent 8. This means, the stent 8 and in particular the slant 17 is designed to match the anatomic edge conditions associated with the branching or bifurcating vessels 1, 3. In other words, the slant plane 19 is defined by the intersection of two cylinders with different diameters representing the parent and branch vessels 1, 3, the slant length L is defined as a distance between the distal edge 33 of the face side 15 and the intersection point 20 between the plane 19 of the ostial circumference and the line drawn through the distal edge 33 of the face side 15 and the most distal point 30 of the ostial circumference 6. The slant angle β is defined as an angle between the face side 15 and the plane 19 of the ostium of the branching vessel 3. The angle β and the length L fully define the part of a hollow stent cylinder protruding into the parent vessel 1 removed by the truncation of a hollow stent cylinder along the slant plane 19. This enables an accurate determination of the stent geometry which simplifies the effort to produce the stent 8. In particular, the slant 17 is designed in such a way that when implanting the stent 8 into the side branch vessel 3 which branches from the parent vessel 1 under a branching angle α, the slant 17 is oriented towards the branching point 5 of the vessels 1, 3 and the end 12 of the stent 8 which comprises the slant plane 19 is flush with the inner surface 23 of the parent vessel 1 corresponding to an ostium of the branching vessels 1, 3. The slant 17 comprises the slant angle β which is disposed between the face side 15 of the stent 8 and the plane 19 of the ostium of the branching vessels 1, 3 and the slant 17 comprises the slant length L which is disposed along the longitudinal axis 18 of the stent 8 and defines a distance between the distal edge 33 of the face side 15 and a most distal point 30 of the ostial circumference 6 and the slant 17 comprises the slant plane 19 comprising the intersection of two cylinders with different diameters representing the two branching vessels 1, 3.

Preferably the stent 8 comprises a first radiopaque marker 21 which is disposed at a most proximal cell 22 of the mesh structure 9. The most proximal cell 22 is preferably arranged at a most proximal section 31 of the slanted end 12. Preferably, the stent 8 further comprises a second marker 28 which is disposed at a most distal cell 29 of the mesh structure 9. The most distal cell 29 is preferably arranged at a most distal section 32 of the slanted end 12. In a preferred embodiment of the stent 8, the stent 8 comprises both the first and the second marker 21, 28, wherein when implanting the stent 8 into the side branch vessel 3 the first radiopaque marker 21 and the second radiopaque marker 28 can be placed such that they match with a line drawn through the most proximal point 7 and the most distal point 30 of the ostial circumference 6 as visualized in orthogonal view on the plane 19. In an alternative embodiment of the stent 8, the stent 8 comprises just one radiopaque marker 21, 28. The stent 8 preferably comprises the mesh-like structure 9 comprising the first radiopaque marker 21 being disposed at the most proximal mesh cell 22 of said stent 8 and the second radiopaque marker 28 being disposed at the most distal mesh cell 29 at the slant plane 19 of the stent 8, wherein the markers 21, 28 can be positioned such that the first radiopaque marker 21 is placed at a most proximal point 7 and/or said second radiopaque marker 28 is placed at the most distal point 30 of the ostial circumference 6 of said branching vessels 1, 3.

In the following, the application of the stent 8 is explained. Initially, the stent 8 is fed to the site where it should be applied, for example to the area of the bifurcation or branching point 5 of the branch vessel 3. To do so, the stent 8 is preferably crimped on an inflatable balloon disposed at the end of a catheter. Alternatively, the stent 8 is self expanding. That means, the stent 8 is made of a material that changes its shape in dependency on the ambient temperature. The slant 17 of the stent 8 is arranged in that way that it is oriented towards the branching point 5 and that the slant 17 is flush with the inner surface 23 of the parent vessel 1. To facilitate this positioning of the slant 17, in a preferred embodiment of the stent 8 the first radiopaque marker 21 is provided at the most proximal mesh cell 22. The first radiopaque marker 21 and the second radiopaque marker 28 are preferably positioned such that they match with the line drawn through the most proximal point 7 and through the most distal point 30 of the ostial circumference 6. This positioning is preferably done when the target site, i.e. the branching area of the vessels 1, 3 is visualized in an orthogonal projection. Due to the matching of the two markers 21, 28 with the most proximal and the most distal points 7, 30 on the projection line of the ostial circumference 6 as visualized in orthogonal view, a malposition of the stent 8 during implantation is reliably avoided. In particular with longer stents 8 two markers 21, 28 are very advantageous to avoid a torque of the stent 8. After that, the stent 8 is expanded to the diameter D8 which corresponds to the diameter D3 of the branch vessel. To ensure that the slant 17 is completely flush with the inner surface 23 of the parent vessel 1 and does not extend into the lumen 2, different slant angles β are used which depend on the branching angle α of the vessels 1, 3. This means, the slant angle β is chosen to match the anatomic edge conditions of the vessels 1, 3. The following table displays exemplary different slant angles β and slant lengths L in dependence on the branching angle α and the diameter D8 of the stent 8 or the diameter D3 of the branch vessel 3, respectively.

| D3; D8 [mm] | α[°] | β [°] | L [mm] |
|---|---|---|---|
| 2 | 30 | 60 | 3.464 |
| | 40 | 50 | 2.384 |
| | 50 | 40 | 1.678 |
| | 60 | 30 | 1.155 |
| | 70 | 20 | 0.728 |
| | 80 | 10 | 0.353 |
| 2,5 | 30 | 60 | 4.330 |
| | 40 | 50 | 2.980 |
| | 50 | 40 | 2.098 |
| | 60 | 30 | 1.443 |
| | 70 | 20 | 0.910 |
| | 80 | 10 | 0.434 |
| 3 | 30 | 60 | 5.196 |
| | 40 | 50 | 3.576 |
| | 50 | 40 | 2.517 |
| | 60 | 30 | 1.732 |
| | 70 | 20 | 1.109 |
| | 80 | 10 | 0.521 |

In addition, the curvature of the space curve depending on the diameter D1 of the parent vessel 1 inscribing the slant plane 19 is also considered.

Given the typical spread and distribution of branching or bifurcation angles α and diameters of the bifurcating vessels 1, 3 a limited number of stents 8, approximately up to twelve would be sufficient to cover the entire range of vascular bifurcations.

After placing the stent 8 in the branch vessel 3 it is further possible to place one or more stents 24 in the parent vessel 1, if needed. Due to the fact that the stent 8 in the branch vessel 3 is flush with the inner surface 23 of the parent vessel 1 the stent 8 arranged in the branch vessel 3 will not collide or negatively interact with the parent vessel stent 24. Consequently, the stent 8 placement can be combined freely with any stent implantation procedures performed on the parent vessel 1. The parent vessel stent 24 comprises a longitudinal axis 25 which might intersect with the longitudinal axis 18.

Although the present invention is described completely by means of preferred embodiments it is not limited to this embodiments but modifiable in various ways. In particular, features of the embodiments described before are combinable in arbitrary manner.

The above mentioned materials, dimensions and numerical data are only exemplary and serve to describe the embodiments and improvements of the invention and have no limiting character.

### Reference Signs

- 1: parent vessel
- 2: lumen
- 3: branch vessel
- 4: lumen
- 5: branching point
- 6: ostial circumference
- 7: most proximal point
- 8: stent
- 9: mesh structure
- 10: cell
- 11: strut
- 12: end section
- 13: end section
- 14: lateral surface
- 15: face side
- 16: face side
- 17: slant
- 18: longitudinal axis
- 19: slant plane
- 20: intersection point
- 21: first radiopaque marker
- 22: proximal mesh cell
- 23: inner surface
- 24: parent vessel stent
- 25: longitudinal axis
- 26: inner surface
- 27: cylindrical stent space
- 28: second radiopaque marker
- 29: distal mesh cell
- 30: most distal point
- 31: proximal section
- 32: distal section
- 33: distal edge
- D1: diameter
- D3: inner diameter
- D8: expanded diameter
- L: slant length
- α: branching or bifurcation angle
- β: slant angle

## Claims

1. Stent (8), in particular for the treatment of cardiovascular diseases, wherein an end (12) of said stent (8) comprises a slant (17).

2. Stent according to claim 1, **characterized in that** said slant (17) truncates a cylindrical stent space (27) which is generated by a lateral surface (14) of said stent (8) at least sectionally.

3. Stent according to claim 1 or 2, **characterized in that** said slant (17) comprises a slant angle (β) disposed between a face side (15) and a slant plane (19) of said stent (8) and said slant (17) comprises a slant length (L) disposed along a longitudinal axis (18) of said stent (8) defining a distance between said face side (15) and an intersection point (20) between said slant plane (19) and a lateral surface (14) of said stent (8).

4. Stent according to claim 3, **characterized in that** said slant angle (β) is between 10° and 60°.

5. Stent according to one of the preceding claims, **characterized in that** said slant (17) is designed in such a way that when implanting said stent (8) into a side branch vessel (3) which branches from a parent vessel (1) under a branching angle (α), said slant (17) is oriented towards a branching point (5) of said vessels (1, 3) and said end (12) of said stent (8) which comprises said slant (17) is flush with an inner surface (23) of said parent vessel (1) and said slant scaffolds an ostial circumference (6) and a lumen (4) of said branch vessel (3).

6. Stent according to one of the preceding claims, **characterized in that** said stent (8) is a balloon-expandable or a self-expanding stent (8).

7. Stent according to one of the preceding claims, **characterized in that** said stent (8) comprises a mesh-like structure (9) comprising a first radiopaque marker (21) being disposed at a most proximal mesh cell (22) of said stent and/or a second radiopaque marker (28) being disposed at a most distal mesh cell (29) of said stent (8).

8. Stent according to claim 7, **characterized in that** when implanting said stent (8) into a side branch vessel (3) said first radiopaque marker (21) can be placed to match a most proximal point (7) and/or said second radiopaque marker (28) can be placed to match a most distal point (30) of an ostial circumference (6) of said branching vessels (1, 3).

9. Stent according to one of the preceding claims, **characterized in that** said stent (8) comprises a metal and/or a polymer material, said stent (8) is of a bioabsorbable or a permanent type, and/or said stent (8) comprises an open or covered stent design.

10. Stent according to one of the preceding claims, **characterized in that** said stent (8) is a drug-eluting stent (8).

11. Method for implanting a stent (8), in particular for the treatment of cardiovascular diseases, wherein an end (12) of said stent (8) comprises a slant (17), the method comprising the following steps:
introducing said stent (8) into a side branch vessel (3) which branches from a parent vessel (1); and
arranging said slant (17) of said stent (8) in such a way that said slant (17) is oriented towards a branching point (5) of said vessels (1, 3) and said slant (17) is flush with an inner surface (23) of said parent vessel (1).

12. Method according to claim 11, **characterized in that** said stent (8) is a balloon-expandable or a self-expanding stent (8) and **in that** that after arranging said slant (17) said stent (8) is expanded.

13. Method according to claim 11 or 12, **characterized in that** said stent (8) comprises a first radiopaque marker (21) being disposed at a most proximal mesh cell (22) of said stent (8) and/or a second radiopaque marker (28) being disposed at a most distal mesh cell (29) of said stent, wherein during dilating and implanting said stent (8) said first radiopaque marker (21) is placed to match a most proximal point (7) and/or said second radiopaque marker (28) is placed to match a most distal point (30) of an ostial circumference (6) of said branching vessels (1, 3).

14. Method according to one of claims 11 to 13, **characterized in that** a further stent (24) is implanted into said parent vessel (1), in particular in the area of said branching point (5) of said vessels (1, 3).

15. Method according to one of claims 10 to 12, **characterized in that** the longitudinal axes (18, 25) of said stents (8, 24) intersect each other.
